# EUROPEAN PATENT SPECIFICATION

(11) **EP 2 315 843 B1**
(45) Date of publication and mention of the grant of the patent: **20.04.2016**
(21) Application number: 09808609.3
(22) Date of filing: 11.08.2009
(51) Int. Cl.: C12Q 1/37, C12R 1/01, G01N 33/569

(54) **ENHANCING ENDOTOXIN DETECTION**
VERSTÄRKTE ENDOTOXINERKENNUNG
AMÉLIORATION DE LA DÉTECTION D'ENDOTOXINES

(30) Priority: 18.08.2008 US 193169
(43) Date of publication of application: 04.05.2011
(73) Proprietor: BioDtech, Inc., Birmingham, AL 35209 (US)
(72) Inventor: PEPE, Michael, G., Birmingham, AL 35203 (US); CHAMPION, Milton, Keith, Hoover, AL 35216 (US)
(74) Representative: Leissler-Gerstl, Gabriele
(86) International application number: PCT/US2009/053392
(87) International publication number: WO 2010/021872

(56) References cited:
- US-A- 3 944 391
- US-B2- 6 849 426
- MICHAEL HULKO ET AL: "Inherent chaperone-like activity of aspartic proteases reveals a distant evolutionary relation to double-[psi] barrel domains of AAA-ATPases", PROTEIN SCIENCE (ACADEMIC PRESS INC), vol. 16, no. 4, 1 April 2007 (2007-04-01), pages 644-653, XP55008251, New York NY USA ISSN: 0961-8368, DOI: 10.1110/ps.062478607
- D. PETCH ET AL.: "Proteinase K Digestion of Proteins Improves Detection of Bacterial Endotoxins by the Limulus Amebocyte Lysate Assay: Application for Endotoxin Removal from Cationic Proteins", ANALYTICAL BIOCHEMISTRY (ACADEMIC PRESS INC), vol. 259, 1998, pages 42-47, XP002277870, New York NY USA
- PETSCH, D. ET AL.: 'Proteinase K digestion of proteins improves detection of bacterial endotoxins by the Limulus amebocyte lysate assay: application for endotoxin removal from cationic proteins.' ANALYTICAL BIOCHEMISTRY vol. 259, no. 1, 15 May 1998, pages 42 - 47, XP002277870
- ZHANG, G. H. ET AL.: 'Differential blocking of coagulation-activating pathway s of Limulus amebocyte lysate.' JOURNAL OF CLINICAL MICROBIOLOGY vol. 32, no. 6, June 1994, pages 1537 - 1541, XP008142469
- DUBOSE, D. A. ET AL.: 'Comparison of plasma extraction techniques in preparat ion of samples for endotoxin testing by the Limulus amoebocyte lysate test.' JOURNAL OF CLINICAL MICROBIOLOGY. vol. 11, no. 1, January 1980, pages 68 - 72, XP008142472
- TSUJI, K. ET AL.: 'Use of magnesium to increase sensitivity of Limulus amoebocyte lysate for detection of endotoxin.' APPLIED ENVIRONMENTAL MICROBIOLOGY. vol. 45, no. 4, April 1983, pages 1342 - 1350, XP008142470
- Anonymous: "Endoprep (TM)", BioDtech Inc Application notes, 28 September 2008 (2008-09-28), 28 September 2008 (2008-09-28), pages 1-10, XP55030412, Birmingham AL 35209 USA Retrieved from the Internet: URL:http://www.biodtechinc.com/doc/prod_en doprep.pdf [retrieved on 2012-06-19]
- Lonza Ltd: "Pyrogene Recombinat Factor C endotoxin Detection Assay", , 1 May 2009 (2009-05-01), pages 1-25, XP055197879, Lonza, Walkersville MD USA
- Lonza Ltd: "Q & A session Lonza Webinar innovations in QC using PyroGene (TM) rFC assay", , 19 January 2011 (2011-01-19), pages 1-4, XP055197878, Lonza, Walkersville MD USA

### SEQUENCE LISTING

<110> BioDtech, Inc. Pepe, Michael
<120> Methods and Kits for Enhancing Endotoxin Detection
<130> Not Yet Assigned
<160> 8
<170> Patent In version 3.5
<210> 1
   <211> 34
   <212> PRT
   <213> Artificial Sequence
<220>
   <223> Sushi 1 Peptide
<400> 1
   Gly Phe Lys Leu Lys Gly Met Ala Arg Ile Ser Cys Leu Pro Asn Gly
   1               5                   10                  15
   Gln Trp Ser Asn Phe Pro Pro Lys Cys Ile Arg Glu Cys Ala Met Val
               20                  25                  30
   Ser Ser
<210> 2
   <211> 59
   <212> PRT
   <213> Artificial Sequence
<220>
   <223> Sushi 2 Peptide
<400> 2
   Glu Cys Ala Met Val Ser Ser Pro Glu His Gly Lys Val Asn Ala Leu
   1               5                   10                  15
   Ser Gly Asp Met Ile Glu Gly Ala Thr Leu Arg Phe Ser Cys Asp Ser
               20                  25                  30
   Pro Tyr Tyr Leu Ile Gly Gln Glu Thr Leu Thr Cys Gln Gly Asn Gly
           35                  40                  45
   Gln Trp Asn Gly Gln Ile Pro Gln Cys Lys Asn
       50                  55
<210> 3
   <211> 34
   <212> PRT
   <213> Artificial Sequence
<220>
   <223> Sushi 3 Peptide
<400> 3
   His Ala Glu His Lys Val Lys Ile Gly Val Glu Gln Lys Tyr Gly Gln
   1               5                   10                  15
   Phe Pro Gln Gly Thr Glu Val Thr Tyr Thr Cys Ser Gly Asn Tyr Phe
               20                  25                  30
   Leu Met
<210> 4
   <211> 34
   <212> PRT
   <213> Artificial Sequence
<220>
   <223> Sushi 1 Delta Peptide
<400> 4
   Gly Phe Lys Leu Lys Gly Lys Ala Lys Ile Ser Cys Leu Pro Asn Gly
   1               5                   10                  15
   Gln Trp Ser Asn Phe Pro Pro Lys Cys Ile Arg Glu Cys Ala Met Val
               20                  25                  30
   Ser Ser
<210> 5
   <211> 34
   <212> PRT
   <213> Artificial Sequence
<220>
   <223> Sushi 3 Delta Peptide
<400> 5
   His Ala Glu His Lys Val Lys Ile Lys Val Lys Gln Lys Tyr Gly Gln
   1               5                   10                  15
   Phe Pro Gln Gly Thr Glu Val Thr Tyr Thr Cys Ser Gly Asn Tyr Phe
               20                  25                  30
   Leu Met
<210> 6
   <211> 34
   <212> PRT
   <213> Artificial Sequence
<220>
   <223> Sushi 4 Peptide
<400> 6
   Arg Ala Glu His Lys Val Lys Lys Ile Val Lys Gln Leu Tyr Gly Gln
   1               5                   10                  15
   Phe Arg Gln Leu Thr Arg Val Thr Arg Thr Cys Ser Arg Phe Leu Arg
               20                  25                  30
   Arg Met
<210> 7
   <211> 24
   <212> PRT
   <213> Artificial Sequence
<220>
   <223> Sushi 5 Peptide
<400> 7
   His Lys Val Lys Lys Ile Val Lys Gln Leu Tyr Arg Ala Glu His Lys
   1               5                   10                  15
   Val Lys Lys Ile Val Lys Gln Leu
               20
<210> 8
   <211> 1019
   <212> PRT
   <213> Carcinoscorpius rotundicauda
<400> 8
   Met Val Leu Ala Ser Phe Leu Val Ser Gly Leu Val Leu Gly Leu Leu
   1               5                   10                  15
   Ala Gln Lys Met Arg Pro Val Gln Ser Lys Gly Val Asp Leu Gly Leu
               20                  25                  30
   Cys Asp Glu Thr Arg Phe Glu Cys Lys Cys Gly Asp Pro Gly Tyr Val
           35                  40                  45
   Phe Asp Ile Pro Val Lys Gln Cys Thr Tyr Phe Tyr Arg Trp Arg Pro
       50                  55                  60
   Tyr Cys Lys Pro Cys Asp Asp Leu Glu Ala Lys Asp Ile Cys Pro Lys
   65                  70                  75                  80
   Tyr Lys Arg Cys Gln Glu Cys Lys Ala Gly Leu Asp Ser Cys Val Thr
                   85                  90                  95
   Cys Pro Pro Asn Lys Tyr Gly Thr Trp Cys Ser Gly Glu Cys Gln Cys
               100                 105                 110
   Lys Asn Gly Gly Ile Cys Asp Gln Arg Thr Gly Ala Cys Ala Cys Ala
           115                 120                 125
   Asp Arg Tyr Glu Gly Val His Cys Glu Ile Leu Lys Gly Cys Pro Leu
       130                 135                 140
   Leu Pro Ser Asp Ser Gln Val Gln Glu Val Arg Asn Pro Pro Asp Asn
   145                 150                 155                 160
   Pro Gln Thr Ile Asp Tyr Ser Cys Ser Pro Gly Phe Lys Leu Lys Gly
                   165                 170                 175
   Met Ala Arg Ile Ser Cys Leu Pro Asn Gly Gln Trp Ser Asn Phe Pro
               180                 185                 190
   Pro Lys Cys Ile Arg Glu Cys Ala Met Val Ser Ser Pro Glu His Gly
           195                 200                 205
   Lys Val Asn Ala Leu Ser Gly Asp Met Ile Glu Gly Ala Thr Leu Arg
       210                 215                 220
   Phe Ser Cys Asp Ser Pro Tyr Tyr Leu Ile Gly Gln Glu Thr Leu Thr
   225                 230                 235                 240
   Cys Gln Gly Asn Gly Gln Trp Asn Gly Gln Ile Pro Gln Cys Lys Asn
                   245                 250                 255
   Leu Val Phe Cys Pro Asp Leu Asp Pro Val Asn His Ala Glu His Lys
               260                 265                 270
   Val Lys Ile Gly Val Glu Gln Lys Tyr Gly Gln Phe Pro Gln Gly Thr
           275                 280                 285
   Glu Val Thr Tyr Thr Cys Ser Gly Asn Tyr Phe Leu Met Gly Phe Asp
   290 295 300
   Thr Leu Lys Cys Asn Pro Asp Gly Ser Trp Ser Gly Ser Gln Pro Ser
   305 310 315 320
   Cys Val Lys Val Ala Asp Arg Glu Val Asp Cys Asp Ser Lys Ala Val
   325 330 335
   Asp Phe Leu Asp Asp Val Gly Glu Pro Val Arg Ile His Cys Pro Ala
   340 345 350
   Gly Cys Ser Leu Thr Ala Gly Thr Val Trp Gly Thr Ala Ile Tyr His
   355 360 365
   Glu Leu Ser Ser Val Cys Arg Ala Ala Ile His Ala Gly Lys Leu Pro
   370 375 380
   Asn Ser Gly Gly Ala Val His Val Val Asn Asn Gly Pro Tyr Ser Asp
   385 390 395 400
   Phe Leu Gly Ser Asp Leu Asn Gly Ile Lys Ser Glu Glu Leu Lys Ser
   405 410 415
   Leu Ala Arg Ser Phe Arg Phe Asp Tyr Val Arg Ser Ser Thr Ala Gly
   420 425 430
   Lys Ser Gly Cys Pro Asp Gly Trp Phe Glu Val Asp Glu Asn Cys Val
   435 440 445
   Tyr Val Thr Ser Lys Gln Arg Ala Trp Glu Arg Ala Gln Gly Val Cys
   450 455 460
   Thr Asn Met Ala Ala Arg Leu Ala Val Leu Asp Lys Asp Val Ile Pro
   465 470 475 480
   Asn Ser Leu Thr Glu Thr Leu Arg Gly Lys Gly Leu Thr Thr Thr Trp
   485 490 495
   Ile Gly Leu His Arg Leu Asp Ala Glu Lys Pro Phe Ile Trp Glu Leu
   500 505 510
   Met Asp Arg Ser Asn Val Val Leu Asn Asp Asn Leu Thr Phe Trp Ala
   515 520 525
   Ser Gly Glu Pro Gly Asn Glu Thr Asn Cys Val Tyr Met Asp Ile Gln
       530                 535                 540
   Asp Gln Leu Gln Ser Val Trp Lys Thr Lys Ser Cys Phe Gln Pro Ser
   545                 550                 555                 560
   Ser Phe Ala Cys Met Met Asp Leu Ser Asp Arg Asn Lys Ala Lys Cys
                   565                 570                 575
   Asp Asp Pro Gly Ser Leu Glu Asn Gly His Ala Thr Leu His Gly Gln
               580                 585                 590
   Ser Ile Asp Gly Phe Tyr Ala Gly Ser Ser Ile Arg Tyr Ser Cys Glu
           595                 600                 605
   Val Leu His Tyr Leu Ser Gly Thr Glu Thr Val Thr Cys Thr Thr Asn
       610                 615                 620
   Gly Thr Trp Ser Ala Pro Lys Pro Arg Cys Ile Lys Val Ile Thr Cys
   625                 630                 635                 640
   Gln Asn Pro Pro Val Pro Ser Tyr Gly Ser Val Glu Ile Lys Pro Pro
                   645                 650                 655
   Ser Arg Thr Asn Ser Ile Ser Arg Val Gly Ser Pro Phe Leu Arg Leu
               660                 665                 670
   Pro Arg Leu Pro Leu Pro Leu Ala Arg Ala Ala Lys Pro Pro Pro Lys
           675                 680                 685
   Pro Arg Ser Ser Gln Pro Ser Thr Val Asp Leu Ala Ser Lys Val Lys
       690                 695                 700
   Leu Pro Glu Gly His Tyr Arg Val Gly Ser Arg Ala Ile Tyr Thr Cys
   705                 710                 715                 720
   Glu Ser Arg Tyr Tyr Glu Leu Leu Gly Ser Gln Gly Arg Arg Cys Asp
                   725                 730                 735
   Ser Asn Gly Asn Trp Ser Gly Arg Pro Ala Ser Cys Ile Pro Val Cys
               740                 745                 750
   Gly Arg Ser Asp Ser Pro Arg Ser Pro Phe Ile Trp Asn Gly Asn Ser
           755                 760                 765
   Thr Glu Ile Gly Gln Trp Pro Trp Gln Ala Gly Ile Ser Arg Trp Leu
   770 775 780
   Ala Asp His Asn Met Trp Phe Leu Gln Cys Gly Gly Ser Leu Leu Asn
   785 790 795 800
   Glu Lys Trp Ile Val Thr Ala Ala His Cys Val Thr Tyr Ser Ala Thr
   805 810 815
   Ala Glu Ile Ile Asp Pro Asn Gln Phe Lys Met Tyr Leu Gly Lys Tyr
   820 825 830
   Tyr Arg Asp Asp Ser Arg Asp Asp Asp Tyr Val Gln Val Arg Glu Ala
   835 840 845
   Leu Glu Ile His Val Asn Pro Asn Tyr Asp Pro Gly Asn Leu Asn Phe
   850 855 860
   Asp Ile Ala Leu Ile Gln Leu Lys Thr Pro Val Thr Leu Thr Thr Arg
   865 870 875 880
   Val Gln Pro Ile Cys Leu Pro Thr Asp Ile Thr Thr Arg Glu His Leu
   885 890 895
   Lys Glu Gly Thr Leu Ala Val Val Thr Gly Trp Gly Leu Asn Glu Asn
   900 905 910
   Asn Thr Tyr Ser Glu Thr Ile Gln Gln Ala Val Leu Pro Val Val Ala
   915 920 925
   Ala Ser Thr Cys Glu Glu Gly Tyr Lys Glu Ala Asp Leu Pro Leu Thr
   930 935 940
   Val Thr Glu Asn Met Phe Cys Ala Gly Tyr Lys Lys Gly Arg Tyr Asp
   945 950 955 960
   Ala Cys Ser Gly Asp Ser Gly Gly Pro Leu Val Phe Ala Asp Asp Ser
   965 970 975
   Arg Thr Glu Arg Arg Trp Val Leu Glu Gly Ile Val Ser Trp Gly Ser
   980 985 990
   Pro Ser Gly Cys Gly Lys Ala Asn Gln Tyr Gly Gly Phe Thr Lys Val
   995 1000 1005
   Asn Val Phe Leu Ser Trp Ile Arg Gln Phe Ile
   1010 1015

## Description

### BACKGROUND

Endotoxin, also known as lipopolysaccharide (LPS), is an integral component of the gram-negative bacterial cell membrane and is responsible for many, if not all, of the toxic effects that occur during gram-negative bacterial sepsis. LPS is a mixture of glycolipids consisting of a variable polysaccharide domain covalently bound to a conserved glueosamine-based phospholipid known as lipid A. LPS directly simulates host monocytcs and macrophages to secrete a wide array of inflammatory cytokincs that include tumor necrosis factor-α (TNF-α), interleukins-1 (IL-1), and interleukin-8 (IL-8). Excessive release of these cytokines by host macrophages contributes to organ failure and death that occur after episodes of gram-negative bacterial sepsis. The pro-inflammatory bioactivities exhibited by LPS typically reside in the lipid A moiety.

### SUMMARY

Provided herein arc methods as defined in the claims for detecting gram negative bacteria or lipopolysaccharide in a sample. For example, provided is a method for detecting gram negative bacteria or Lipopolysaccharide in a sample comprising contacting the sample with one or more lipopolysaccharide binding polypeptides in the presence of an inactive acidic protease, and determining whether the one or more lipopolysaccharide binding polypeptides bind to the sample. Binding indicates the sample contains gram negative bacteria or lipopolysacoharide. Also provided herein arc methods for detecting gram negative bacteria or lipopolysaccharide in a sample comprising contacting the sample with an active acidic protease under conditions that result in degradation of the proteins in the sample, contacting the sample with one or more lipopolysaccharide binding polypeptides in the presence of an inactive acidic protease, and determining whether the one or more lipopolysaccharide binding polypeptides bind to the sample.

Provided is a method for detecting gram negative bacteria or lipopolysaccharide in a sample comprising cantacting the sample with an active acidic protease under conditions that result in degradation of the proteins in the sample, further contacting the sample with an amebocyte lysate, and determining whether a gelation reaction occurs in the amebocyte lysate.

The use of hits for detecting gram negative bacteria or lipopolysaccharide in a sample is disclosed. The kits comprise an acidic protease. The kits also comprise one or more amebocyte lysates and/or one or more lipopolysaccharide binding polypeptides.

The details of one or more aspects are set forth in the accompanying drawings and description below. Other features, objects, and advantages will be apparent from the description and drawings, and from the claims.

### DESCRIPTION OF DRAWINGS

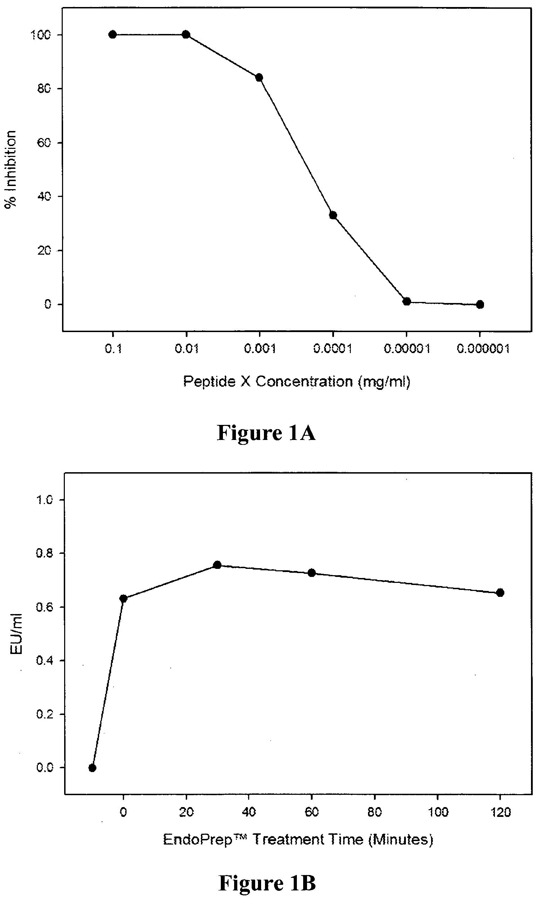

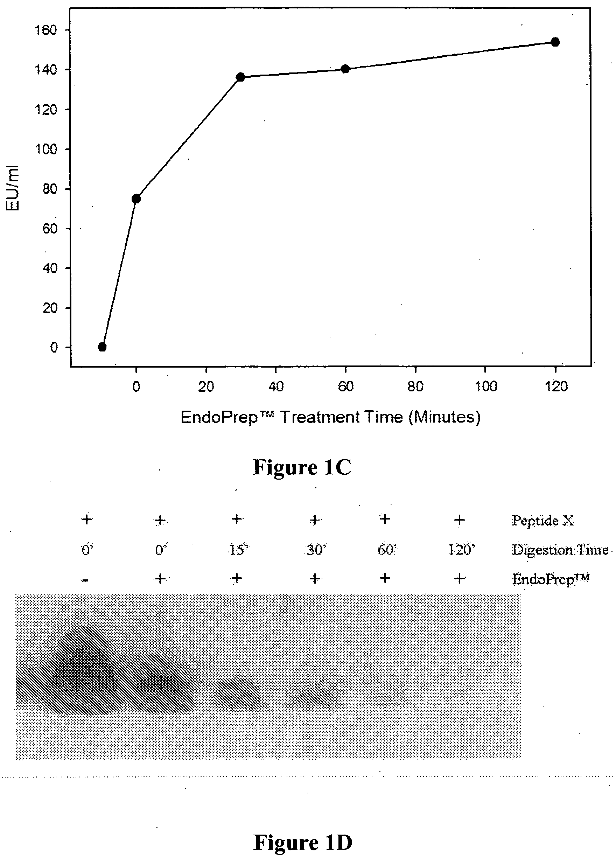

Figures 1A-1D show treatment of Peptide X with an EndoPrep™ protease composition (BioDtech, Inc., Birmingham, AL). Figure 1A is a graph of a PPC inhibition assay demonstrating the extent of peptide dilution required to overcome the inhibitory effect of Peptide X in endotoxin detection. Figure 1B is a graph demonstrating the recovery of a 1 EU/ml PPC in samples of Peptide X before and after treatment with an EndoPrep™ protease composition. Figure 1C is a graph demonstrating the recovery of defined endotoxin contamination in a sample of Peptide X. Figure 1D is an image of a PAGE gel showing Peptide X degradation with an EndoPrep™ protease composition treatment.

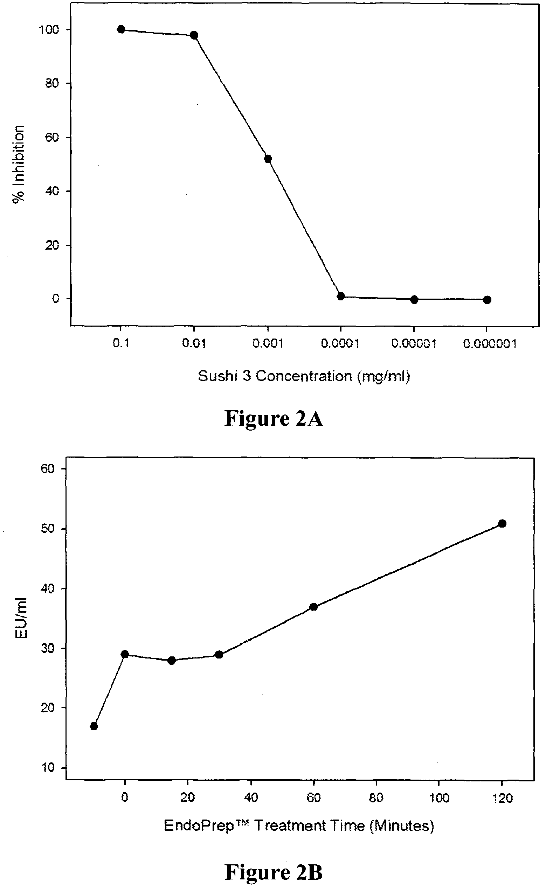

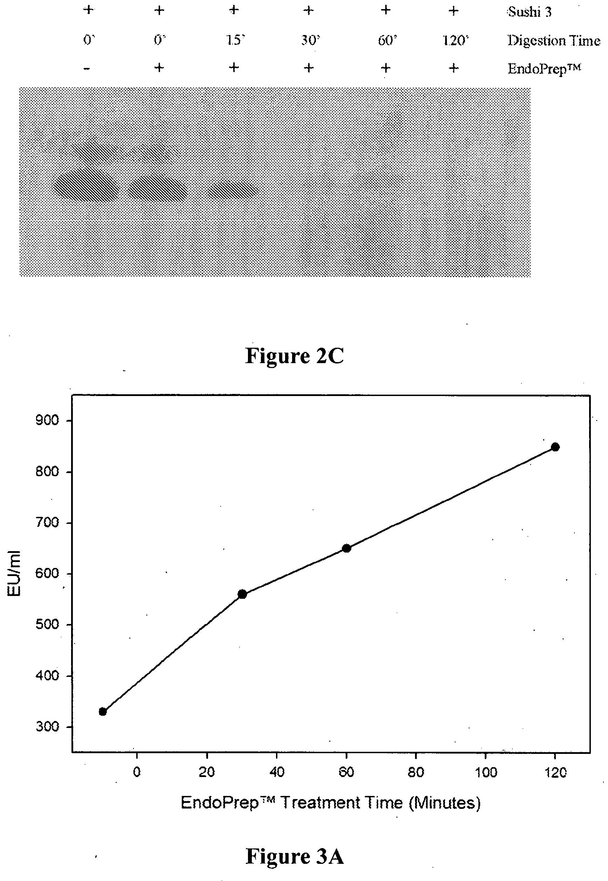

Figures 2A-2C show the treatment of Sushi 3 peptide with an EndoPrep™ protease composition. Figure 2A is a graph of a PPC inhibition assay demonstrating the extent of peptide dilution required to overcome the inhibitory effect of Sushi3 peptide in endotoxin detection. Figure 2B is a graph demonstrating the recovery of a defined endotoxin contamination in a sample of Sushi 3 Peptide. Figure 2C is an image of a PAGE gel demonstrating Sushi 3 Peptide degradation with an EndoPrep™ treatment protease composition.

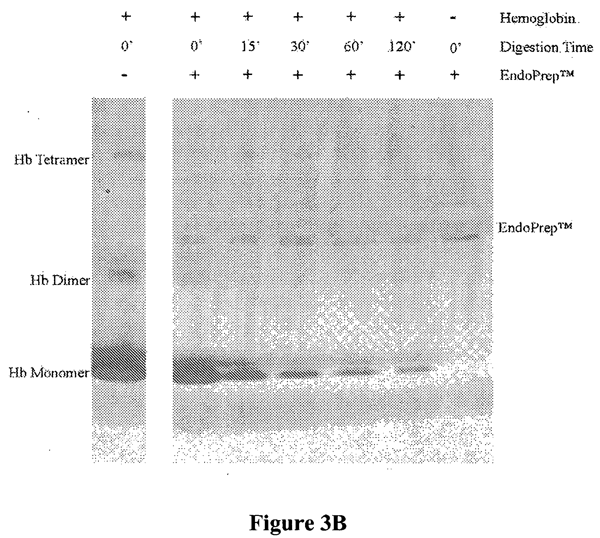

Figures 3A-3B show the treatment of Hemoglobin with an EndoPrep™ protease composition. Figure 3A is a graph demonstrating the recovery of endogenous endotoxin contamination in a sample of hemoglobin from bovine erythrocytes. Figure 3B is an image of a PAGE gel demonstrating hemoglobin degradation with an EndoPrep™ protease composition treatment. The gel was run under non-reducing conditions resulting in three distinct hemoglobin populations. Locations of each hemoglobin population and the EndoPrep™ protease are indicated.

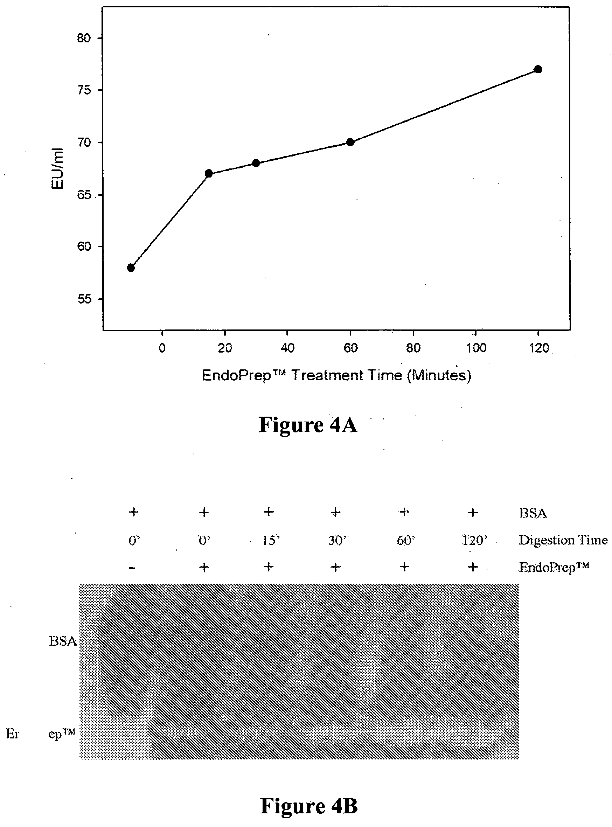

Figures 4A-4B show the treatment of BSA with an EndoPrep™ protease composition. Figure 4A is a graph demonstrating the recovery of a defined endotoxin contamination in a sample of BSA. Figure 4B is an image of a PAGE gel demonstrating BSA degradation with an EndoPrep™ protease composition treatment. Location of BSA and the EndoPrep™ protease are indicated.

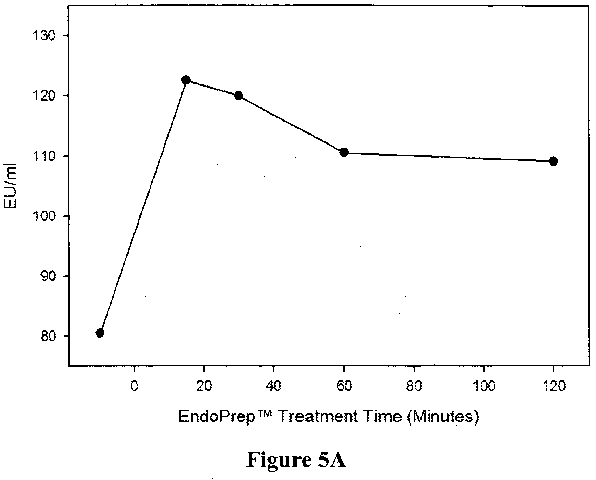

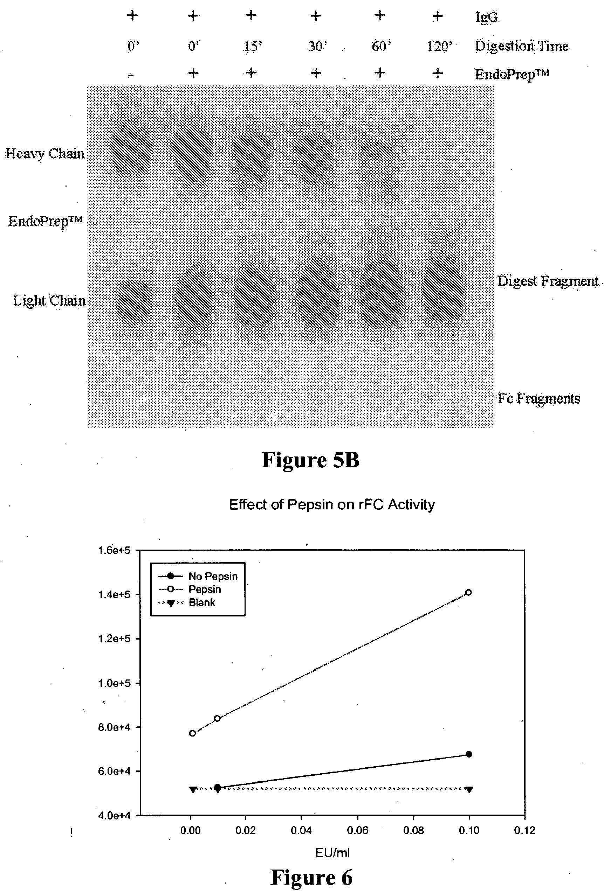

Figures 5A-5B show the treatment of IgG with an EndoPrep™ protease composition. Figure 5A is a graph demonstrating the recovery of a defined endtoxin contamination in a sample of rabbit IgG from plasma. Figure 5B is an image of a PAGE gel demonstrating IgG degradation with an EndoPrep™ protease composition treatment. The gel was run under reducing conditions resulting in the separation of the light and heavy chains. Digestion results in the cleavage of the heavy chain into a large fragment and small Fc fragments. Locations of the heavy and light chains, the EndoPrep™ protease, and the digestion products are indicated.
Figure 6 is a graph showing the effect of pepsin on recombinant Factor C (rFC) activity. Data shown are from the 60 minute PyroGene® Lonza (Walkersville, MD) incubation. Solid black line with closed black circles for data points indicates results from LPS in water. Solid black line with open circles for data points indicates results from LPS in water containing 0.05% pepsin. Dotted black line with triangles for data points indicates the value of water. Treatment with pepsin enhances the activity of the rFC Enzyme as evidenced by the increase in fluorescene.

### DETAILED DESCRIPTION

Since endotoxin contains a negative charge, a lipid and a carbohydrate, many proteins are known to bind endotoxin and affect the ability of assays to properly measure it. Specifically, proteins binding to endotoxin can cause either inhibition or enhancement in the standard assays resulting in false positives and negatives, as well as affecting accuracy. For example, serine protease inhibitors, such as, soybean trypsin inhibitor, alpha2 macroglobulin, aprotinin, anti-plasmin, anti-thrombin III, anti-trypsin and hirudin, inhibit methods of detecting endotoxin. As described in the examples below, treatment of such samples, including serum and blood samples, with an acidic protease degrades the proteins in the sample without affecting endotoxin, thus, increasing the accuracy of endotoxin detection assays. The acidic proteases can be used in, for example, LAL, Factor C and cytokine ELISA assays. There are a variety of assays for the diagnosis of gram negative bacterial infections. The current assay used by pharmaceutical and medical industries is based on a substance called Limulus Amebocyte Lysate (LAL). The lysate contains proteins that react in the presence of endotoxins.

Factor C is a serine protease zymogen. It is the key enzyme in the *C*. *rotundicauda* amebocyte lysate (CAL) that is activated by LPS to initiate the coagulation cascade. Factor C activity is the basis of a sensitive assay for femtogram levels of endotoxin used in the quality control of pharmaceutical products. The importance of Factor C in the detection of endotoxin has led to the expression of recombinant Factor C, rFC, as an alternative source to alleviate the batch-to-batch and seasonal variation in the sensitivity of detection of endotoxin. s

Inactive acidic proteases, such as inactive pepsin and inactive HIV protease, enhance the activity of Factor C by acting as a chaperone and maintaining Factor C in proper conformation. The ability of inactive pepsin and inactive HIV protease to act as chaperones has been described (Hulko et al., Protein Science 16:644-53 (2007)).

Provided herein arc improved methods and the use of kits as defined in the claim for detecting gram negative bacteria or lipopolysaccharide in a sample. For example, provided herein are methods for detecting gram negative bacteria or lipopolysaccharide in a sample comprising contacting the sample with one or more lipopolysaccharide binding polypeptides in the presence of an inactive acidic protease, and determining whether the one or more lipopolysaccharide binding polypeptides bind to the sample. Binding indicates the sample contains gram negative bacteria or lipopolysaccharide. Optionally, the method further comprises inactivating the acidic protease prior to contacting the sample with the one or more lipopolysaccharide binding polypeptides. Optionally, the method further comprises contacting the sample with an active acidic protease under conditions that result in degradation of the proteins in the sample prior to contacting the sample with the one or more lipopolysaccharide binding polypeptides. Provided herein arc methods for detecting gram negative bacteria or lipopolysaccharide in a sample comprising contacting the sample with an active acidic protease under conditions that result in degradation of the proteins in the sample, contacting the sample with one or more lipopolysaccharide binding polypeptides in the presence of an inactive acidic protease, and determining whether the one or more lipopolysaccharide binding polypeptides bind to the sample. Binding indicates the sample contains gram negative bacteria or lipopolysaccharide. The method further comprises inactivating the acidic protease after contacting the sample with the active acidic protease. Optionally, a digestion buffer comprises the active acidic protease. Optionally, the acidic protease is inactivated by a pH of about 7.0.

Also provided is a method for detecting gram negative bacteria or lipopolysaccharide in a sample comprising contacting the sample with an active acidic protease under conditions that result in degradation of the proteins in the sample, further contacting the sample with an amebocyte lysate, and determining whether a gelation reaction occurs in the amebocyte lysate. A gelation reaction indicates the sample contains gram negative bacteria or lipopolysaccharide. Optionally, the amebocyte lysate is selected from the group consisting of lysates of *Limulus polyphemus, Tachypleus tridentatus, Carcinoscorpius rotundicauda* and *Tachypleus gigas.*

Lipopolysaccharide binding polypeptides suitable for use in the provided methods and kits include lipopolysaccharide binding polypeptides of an amebocyte lysate. The amebocyte lysate is, optionally, selected from the group consisting of lysates of *Limulus polyphemus, Tachypleus tridentatus, Carcinoscorpius rotundicauda* and *Tachypleus gigas.* Optionally, the one or more lipopolysaccharide binding polypeptides are polypeptides comprising a lipopolysaccharide binding domain of a Factor C protein. The endotoxin/lipid A-binding domain of Factor C lies within the amino terminal portion of the protein encompassed by rFCES; that is, the first 350 amino acids, numbered as in SEQ ID NO:8. The Factor C protein and its domains, including sushi domains, are described in U.S. Patent No. 6,719,973. U.S. Patent No. 6,719,973 also discloses sushi peptides of the Factor C protein and methods for making and using the sushi peptides. Thus, the lipopolysaccharide binding domain of a Factor C protein is optionally a sushi domain or peptide. The lipopolysaccharide binding domain of a Factor C protein is, for example, amino acids 1-333 of a Factor C protein (e.g., amino acids 1-333 of SEQ ID NO:8), amino acids 29-330 of SEQ ID NO:8, a sushi 1 domain of a Factor C protein (e.g., amino acids 29-201 of SEQ ID NO:8), a sushi 2 domain of a Factor C protein (e.g., amino acids 195-260 of SEQ ID NO:8), a sushi 3 domain of a Factor C protein (e.g., amino acids 264-330 of SEQ ID NO:8), a sushi 1 peptide (SEQ ID NO:1), a sushi 2 peptide (SEQ ID NO:2); a sushi 3 peptide (SEQ ID NO:3), a sushi 1Δ peptide (SEQ ID NO:4), a sushi 3Δ peptide (SEQ ID NO:5), a sushi 4 peptide (SEQ ID NO:6) or a sushi 5 peptide (SEQ ID NO:7).

Optionally, the step of contacting the sample with the one or more lipopolysaccharide binding polypeptides comprises contacting the sample with a dimer or a tetramer comprising a lipopolysaccharide binding domain of a Factor C protein. As used herein, the phrase Factor C dimer refers to two molecules of a Factor C protein or portion thereof linked together. The provided Factor C dimers comprise a first and a second polypeptide, wherein the first and second polypeptides each comprise a lipopolysaccharide binding domain of a Factor C protein. Optionally, the first and second polypeptide are the same or different polypeptides. Optionally, the lipopolysaccharide binding domain of a Factor C protein is a sushi peptide. The sushi peptide can be a sushi 1 peptide (SEQ ID NO:1), a sushi 2 peptide (SEQ ID NO:2), a sushi 3 peptide (SEQ ID NO:3), a sushi 1Δ peptide (SEQ ID NO:4), a sushi 3Δ peptide (SEQ ID NO:5), a sushi 4 peptide (SEQ ID NO:6) or a sushi 5 peptide (SEQ ID NO:7). Optionally, the sushi peptide is a sushi 3 peptide or a sushi 3Δ peptide. Optionally, the polypeptides of the Factor C dimer are linked by disulfide bonds. A disulfide bond is a bond between cysteine residues. Optionally, each of the first and second polypeptides that comprise the Factor C dimer consists of SEQ ID NOs:1, 2, 3, 4, 5, 6 or 7.

The term protein, peptide, polypeptide, or peptide portion is used broadly herein to mean two or more amino acids linked by a peptide bond. It should be recognized that the terms peptide and polypeptide are not used herein to suggest a particular size or number of amino acids comprising the molecule and that a peptide can contain up to several amino acid residues or more. The provided peptides are produced by a proteolytic reaction on a polypeptide, i.e., a peptide produced upon cleavage of a peptide bond in the polypeptide. The provided peptides are optionally produced using methods of chemical synthesis or methods of recombinant DNA technology, to produce a synthetic polypeptide. For example, the provided peptides are synthesized using stepwise solid phase peptide synthesis on 2-chlorotrityl resin using standard Fmoc protecting groups and pseudo-prolines. Alternatively, the peptides are synthesized by combining two or more smaller peptides that have been chemically synthesized.

Nucleic acids that encode the aforementioned peptide sequences, variants and fragments thereof are also disclosed. These sequences include all degenerate sequences related to a specific polypeptide sequence, i.e., all nucleic acids having a sequence that encodes one particular polypeptide sequence as well as all nucleic acids, including degenerate nucleic acids, encoding the disclosed variants and derivatives of the polypeptide sequences. Thus, while each particular nucleic acid sequence may not be written out herein, it is understood that each and every sequence is in fact disclosed and described herein through the disclosed protein sequence. A wide variety of expression systems are used to produce Factor C peptides as well as fragments, isoforms, and variants.

The nucleic acid sequences provided herein are examples of the genus of nucleic acids and are not intended to be limiting. Also provided are expression vectors comprising nucleic acids that encode the peptide sequences, variants or fragments thereof, wherein the nucleic acids are operably linked to an expression control sequence. Further provided are cultured cells comprising the expression vectors. Such expression vectors and cultured cells can be used to make the provided peptides.

By isolated or purified is meant a composition (e.g., a polypeptide or nucleic acid) that is substantially free from other materials, including materials with which the composition is normally associated in nature. The polypeptides of the invention, or fragments thereof, are obtained, for example, by extraction from a natural source, by expression of a recombinant nucleic acid encoding the polypeptide (e.g., in a cell or in a cell-free translation system), or by chemically synthesizing the polypeptide.

In the provided methods and kits, the lipopolysaccharide binding polypeptides are optionally labeled with a detectable moiety or further comprise a reporter protein or affinity tag. The lipopolysaccharide binding polypeptides are directly labeled or indirectly labeled (e.g., by a secondary or tertiary antibody that is labeled with a detectable moiety). Numerous labels are available including, but not limited to radioisotopes, fluorescent labels, and enzyme-substrate labels. Radioisotopes include, for example, ³⁵S, ¹⁴C, ¹²⁵I, ³H, and ¹³¹I. Fluorescent labels include, for example, rare earth chelates (europium chelates), fluorescein and its derivatives, rhodamine and its derivatives, dansyl, Lissamine, phycoerythrin and Texas Red. The labels are optionally conjugated to the antigen binding partner using the techniques disclosed in Current Protocols in Immunology, Volumes 1 and 2, Coligen et al., Ed., Wiley-Interscience, New York, N.Y., Pubs., (1991), for example.

When using enzyme-substrate labels, the enzyme preferably catalyses a chemical alteration of the chromogenic substrate, which can be measured using various techniques. For example, the enzyme catalyzes a color change in a substrate, which can be measured spectrophotometrically. Alternatively, the enzyme alters the fluorescence or chemiluminescence of the substrate. The chemiluminescent substrate becomes electronically excited by a chemical reaction and then emits light which can be measured (using a chemiluminometer, for example), or donates energy to a fluorescent acceptor. Examples of enzymatic labels include luciferases (e.g., firefly luciferase and bacterial luciferase), luciferin, 2,3-dihydrophthalazinediones, malate dehydrogenase, urease, peroxidase such as horseradish peroxidase (HRP), alkaline phosphatase, β-galactosidase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes are described in O'Sullivan et al., Methods for the Preparation of Enzyme-Antibody Conjugates for use in Enzyme Immunoassay, in Methods in Enzym. (ed J. Langone & H. Van Vunakis), Academic press, New York, 73: 147-166 (1981). Examples of enzyme-substrate combinations include, for example, horseradish peroxidase (HRP) with hydrogen peroxidase as a substrate, alkaline phosphatase (AP) with para-Nitrophenyl phosphate as chromogenic substrate, and β-D-galactosidase (β-D-Gal) with a chromogenic substrate (e.g. p-nitrophenyl-β-D-galactosidase) or fluorogenic substrate 4-methylumbelliferyl-β-D-galactosidase.

An assay is optionally employed wherein the lipopolysaccharide binding polypeptides are attached to a solid support, such as, for example, a column, chip, or plate. The solid support is optionally a mobile solid support (e.g., a bead). A sample derived from the subject or control is passed over (i.e., contacted with) the solid support. Labeled lipopolysaccharide binding polypeptides are then added to the solid support. The amount of label detected that is attached to the target is correlated to a quantity of target in the sample. For example, a lipopolysaccharide binding polypeptide is immobilized on the bottom of a microtiter plate. A sample to be tested is then added to the microtiter plate under conditions that allow binding of the lipopolysaccharide binding polypeptide to gram negative bacteria, endotoxin or lipopolysaccharide if present in the sample. A second lipopolysaccharide binding polypeptide that is labeled with, for example, biotin, is added to the microtiter plate. The labeled polypeptide is detected with avidin coupled to alkaline phosphatase. The avidin coupled to alkaline phosphatase detects bound and labeled polypeptide by liberating p-nitrophenol from a p-nitrophenylphosphate (pNA) substrate producing a yellow color that can be measured at 405 nm.

FACS sorting is used to detect and quantitate, for example, fluorescently labeled beads. For example, the lipopolysaccharide binding polypeptide is linked to a bead. The beads are then contacted with a sample to be tested. A second lipopolysaccharide binding polypeptide that is labeled with, for example, a fluorescent label is added to the beads that have been contacted with the test sample. The bound, labeled beads are then detected and quantitated using a FACS sorter on the basis of one or more fluorescent labels. Optionally, the beads are magnetic beads or fluorescent beads. For example, the lipopolysaccharide binding polypeptide is linked to a mobile solid support such as a bead, which can be added to a biological sample, such as whole blood or blood products like serum, and removed by centrifugation. Optionally, the lipopolysaccharide binding polypeptide is linked to a magnetic bead, such as an iron bead, and removed by a magnetic field.

By way of another example, the lipopolysaccharide binding polypeptide is immobilized on an immobile solid support, such as, for example, a column, filter or membrane. Thus, the lipopolysaccharide binding polypeptide is immobilized, for example, on a hemodialysis membrane or filtration system to remove bacteria and/or endotoxin from whole blood or blood products. Thus, support as used herein refers to any support matrix, such as those solid supports known in the art, which serve to immobilize the lipopolysaccharide binding polypeptide. Suitable supports include, but are not limited to, glass, agarose, plastic, silica, polyacrylamide, hydrogels, gels, membranes, filters, meshes, beads or particles comprised of or coated with cellulose, acrylates, polyacrylates, polyhydroxymethacrylates, polystyrene, dextran, agarose, polysaccharides, hydrophilic vinyl polymers, polymerized derivatives, as well as any porous or non-porous matrices.

Optionally, the lipopolysaccharide binding polypeptides further comprise a reported protein or affinity tag: Suitable reporter proteins include, for example, green fluorescent protein (GFP), alkaline phosphatase, peroxidase and luciferase. Affinity tags include, but are not limited to, polyhistidine, avidin, streptavidin and biotin.

The sample is optionally a biological sample. As used herein, a biological sample subjected to testing is a sample derived from a subject such as a mammal or human and includes, but is not limited to, any biological fluid, including a bodily fluid. Examples of bodily fluids include, but are not limited to, whole blood, serum, urine, saliva, tissue infiltrate, pleural effusions, lung lavage fluid, and the like. The biological fluid includes a cell culture medium or supernatant of cultured cells. For example, the sample can be a blood sample or a serum sample. Optionally, the sample is a liquid sample, such as water or other agents used, for example, in research or clinical laboratories or hospitals. Optionally, the sample is an environmental sample including, but not limited to, fluid, waste, water and rain samples. Optionally, the environmental sample is obtained from a surface, for example, in a hospital, for analysis in the provided methods. For example, a sample can be obtained from a device used in a hospital, clinical or laboratory setting and analyzed for the presence of gram negative bacteria. Optionally, the sample is diluted in solution prior to analysis. Optionally, the sample to be tested comprises a polypeptide in need of degradation. Thus, for example, the sample comprises a polypeptide that inhibits standard endotoxin assays, for example, a serine protease inhibitor.

As used herein, acid, acidic, aspartic or aspartic acid proteases refer to proteases active at low pH. For example, the protease is active at a pH from about 0.0 to about 6.0 or any pH between 0.0 and 6.0, inclusive. Such proteases are inactive at a pH of about 6.0 to about 14.0. As used herein, an inactive acidic protease refers to a protease without protealytic activity (i.e., a protease that is unable to cleave an amino acid sequence such as a polypeptide or protein). As used herein, an active acidic protease refers to a protease with protealytic activity (i.e., a protease that is able to cleave an amino acid sequence). By way of example, an active acidic protease can be inactivated by a pH of 6.5 or higher (i.e., the protease is in a solution with a pH of 6.5 or higher). The pH of a solution can be altered by addition of chemicals to a solution. For example, hydrochloric acid can be used to reduce pH and sodium hydroxide can be used to raise pH. Phosphoric acid can be used to maintain a pH of about 6.5: Optionally, a pepsin inhibitor is used to inactivate pepsin. Pepsin inhibitors include, but are not limited to, acetamidine, N-acetyl-D-phenyalanyl-L-diiodotyrosine, N-acetyl-L-phenyalanyl-D-phenylalaine, p-aminobenzamidine, benzamidine, butyamine, diazoketones, ethylamine, pepstatin, and phenylactamidine.

Acid or acidic proteases, such as endopeptidases, are known and have been grouped into three families, namely, pepsin (A1), retropepsin (A2), and enzymes from pararetroviruses (A3). The members of families A1 and A2 are known to be related to each other, while those of family A3 show some relatedness to A1 and A2. Microbial acid proteases exhibit specificity against aromatic or bulky amino acid residues on both sides of the peptide bond, which is similar to pepsin, but their action is less stringent than that of pepsin. Acid proteases include microbial, fungal, viral, animal and plant acidic proteases. Microbial aspartic proteases can be broadly divided into two groups, (i) pepsin-like enzymes produced by *Aspergillus, Penicillium, Rhizopus,* and *Neurospora* and (ii) rennin-like enzymes produced by *Endothia* and *Mucor* spp (Rao et al., Microbiology and Molecular Biology 62(3):597-635 (1998); Richter et al., Biochem. J. 335:481-90 (1998)). Examples of acidic proteases include, but are not limited to, pepsins, including pepsins A, B and C; rennin; chymosin; plasmepsin; cathepsins, such as, for example, cathepsin D and cathepsin E; human urinary acid protease; and viral proteases like HIV protease. Fungal proteases include, but are not limited to, fungal proteases derived from *Neurospora oryzae*, *Mucor pusillus, Mucor miehei, Aspergillus niger, Rhizopus chinensis,* or *Endothia parasitica*. Microbial proteases include, but are not limited to, yeast proteinase A, aspergillopepsinogen, rhizopuspepsin, penicillopepsin, and endothiapepsin.

Examples 1-6 demonstrate that acidic proteases can be used to enhance endotoxin detection in samples containing molecules that normally inhibit endotoxin detection. The molecules that normally inhibit endotoxin detection include a variety of different classes of peptides, such as short cationic peptides (i.e., Peptide X), enzymes (i.e., Sushi 3 peptide), metalloproteins (i.e., hemoglobin), circulatory proteins (i.e., albumin), and immunoglobulins (i.e., IgG). The examples included whole proteins and small peptides. Additionally, the detection of endogenous and exogenous endotoxin was demonstrated. In all examples shown, treatment with an EndoPrep™ protease composition increased the accuracy of endotoxin detection.

Kits for detecting gram negative bacteria or lipopolysaccharide are provided. The kits comprise an acidic protease. The kits also comprise one or more amebocyte lysates and/or one or more lipopolysaccharide binding polypeptides. Optionally, the lipopolysaccharide binding polypeptides are lipopolysaccharide binding polypeptides of an amebocyte lysate. Optionally, the amebocyte lysate is selected from the group consisting of lysates of *Limulus polyphemus, Tachypleus tridentatus, Carcinoscorpius rotundicauda* and *Tachypleus gigas.* As discussed above; the acidic protease can be any acidic protease. For example, the acidic protease is selected from the group consisting of pepsin, rennin, chymosin, plasmepsin, cathepsin D, cathepsin E, human urinary acid protease, HIV protease, *Neurospora oryzae* protease, *Mucor pusillus* protease, *Mucor miehei* protease, *Aspergillus niger* protease, *Rhizopus chinensis* protease, *Endothia parasitica* protease, yeast proteinase A, aspergillopepsinogen, rhizopuspepsin, penicillopepsin, and endothiapepsin. As also discussed above, the one or more lipopolysaccharide binding polypeptides are, for example, polypeptides comprising a lipopolysaccharide binding domain of a Factor C protein. The kits optionally comprise solid supports such as, for example, a column, plate, chip, or mobile solid support. The mobile solid support is, for example, a magnetic or fluorescent bead. Optionally, the kits further comprising one or more buffers, such as, for example, a digestion buffer. Optionally, the one or more amebocyte lysates and acidic protease are in the same or separate containers. Optionally, the one or more lipopolysaccharide binding polypeptides and the acidic protease are in the same or separate containers. Optionally, the acidic protease is inactive. Optionally, the kits comprise an active acidic protease and an inactive acidic protease, wherein the active acidic protease is the same as or different from the inactive acidic protease. Thus, for example, if the proteases are different, the active protease is pepsin and the inactive protease is chymosin.

Disclosed are materials, compositions, and components that can be used for, can be used in conjunction with, can be used in preparation of, or are products of the disclosed methods and compositions. These and other materials are disclosed herein, and it is understood that when combinations, subsets, interactions, groups, etc. of these materials are disclosed that, while specific reference to each various individual and collective combinations and permutation of these compounds may not be explicitly disclosed, each is specifically contemplated and described herein. For example, if a method is disclosed and discussed and a number of modifications can be made to materials used in the method or in the steps of the method, each and every combination and permutation of the method and the modifications that are possible are specifically contemplated unless specifically indicated to the contrary. Likewise, any subset or combination of these is also specifically contemplated and disclosed. Thus, if there is a variety of additional steps that can be performed in a method, it is understood that each of these additional steps can be performed with any specific method steps or combination of method steps of the disclosed methods, and that each such combination or subset of combinations is specifically contemplated and should be considered disclosed. Thus, a number of aspects have been described. Nevertheless, it will be understood that various modifications may be made. Furthermore, when one characteristic or step is described, it can be combined with any other characteristic or step herein even if the combination is not explicitly stated. Accordingly, all combination of disclosed agents, steps and characteristics are provided even in the absence of explicit disclosure herein.

Ranges may be expressed herein as from about one particular value, and/or to about another particular value. When such a range is expressed, this includes from the one particular value and/or to the other particular value. Similarly, when values are expressed as approximations, by use of the antecedent about, it will be understood that the particular value is disclosed.

The following examples are put forth so as to provide those of ordinary skill in the art with a complete disclosure and description of how the compounds, compositions, articles, devices and/or methods claimed herein are made and evaluated, and are intended to be purely exemplary and are not intended to limit the scope of the methods and materials provided herein.

### Examples

### General Methods

### Materials

Endotoxin detection and quantitiation was performed with the PyroGene® assay from Lonza (Walkersville, MD) according to manufacturer's specifications with and without 1 EU/ml positive product controls (PPC) to validate assay reliability. The assay has a range of detection from 0.001 to 10 EU/ml. Hemoglobin (bovine erythrocytes) was purchased from Calbiochem (La Jolla, CA) and contained endogenous endotoxin at a level of 300 EU/mg. Rabbit IgG (from plasma) was also from Calbiochem and contained approximately 2 EU/mg. Bovine serum albumin (BSA) was purchased from Sigma (St. Louis, MO) and contained <1 EU/mg. Sushi 3 peptide was chemically synthesized by American Peptide (Sunnyvale, CA) and contained no detectable endotoxin. Peptide X was supplied by a proprietary source and contained <1 EU/mg. Endotoxin-free water was from BioDtech, Inc. (Birmingham, AL). When sample endotoxin was low, endogenous endotoxin was added to the levels indicated. Endotoxin was purchased from List Biological Laboratories, Inc. (Campbell, CA) in the form of *Escheria coli O55:B5* lipopolysaccharide.

### EndoPrep™ Protocol

The EndoPrep™ kit consistes of BDTI™ Digestion Buffer (20mM sodium acetate, pH 4.5) (BioDtech, Inc., Birmingham, AL) and EndoPrep™ protease composition (5% pepsin stock solution in BDTI™ Digestion Buffer). All experiments were performed with the following protocol. The protein sample was diluted to a suitable working level using BDTI™ Digestion Buffer. Aliquots of 270 µl of this solution were mixed with 30 µl of the BDTI™ Protease Solution and vortexed for 10 seconds. A sample containing 30 µl of BDTI™ Digestion Buffer instead of the BDTI™ Protease Solution was included in each set of experiments to determine baseline endotoxin without digestion. After mixing, the tubes were covered with Parafilm and incubated in a 37°C water bath for the indicated times. After treatment, the samples were diluted 1:100 in endotoxin-free water and subjected to PyroGene® testing. The 10% sample dilution resulting from addition of BDTI™ Protease Solution was taken into account and calculated into the results given.

### Polyacrylamide Gel Electrophoresis (PAGE)

PAGE analysis was performed with each sample treatment to monitor correlation of endotoxin detection with protein degradation. For PAGE analysis, 20 µl of the undiluted digestion sample was added to a mixture containing 45 µl endotoxin-free water, 10 µl 5 mM DTT, and 25 µl CBS Scientific ClearPAGE™ (Del Mar, CA) 4x Sample Buffer (additional water replaced DTT for non-reducing electrophoresis). This sample was heated in a 70°C water bath for 10 minutes and 17 µl was loaded into a CBS Scientific ClearPAGE 10-20% TEO-CI SDS Gel submerged in CBS Scientific ClearPAGE 1x Tris-Tricine-SDS Run Buffer (Reducing or Non-Reducing) and electrophoresed at 200 Volts for 45 minutes with a current gradient from 60 to 30 mA. All gels were silver stained using Sigma ProteoSilver™ (St. Louis, MO) Silver Stain Kit according to manufacturer's specifications.

### PPC Inhibition Assay

PPC inhibition assays were performed with cationic peptides to determine the extent of inhibition on PPC recovery. Serial 10-fold dilutions of peptide were made in endotoxin-free water. Aliquots of 270 µl of each dilution were added to an endotoxin-free glass tube containing 30 µl of 10 EU/ml LPS for a final endotoxin concentration of 1 EU/ml. 50 µl of each sample was tested in the Lonza PyroGene® (Walkersville, MD) assay. A sample without peptide was included as control and reference.

### PyroGent® Gel Clot Assay

To verify the applicability of the EndoPrep™ protease composition to gel clot assays, digested samples were tested using the Lonza PyroGent® system (Walkersville, MD). For the assay, 100 µl samples were mixed with 100 µl of PyroGent® assay lysate in endotoxin-free glass vials and vortexed for 10 seconds. The samples were then incubated in a 37°C water bath without agitation for 60 minutes. After incubation the samples were tested for clot formation by visual inspection. Along with the samples a series of standards ranging from 0.03 to 0.12 EU/ml and blanks were tested in triplicate to verify assay reliability.

### Example 1: Peptide X

Peptide X (proprietary) is a mixture of short cationic peptides shown to be extremely effective in a therapeutic setting. This product has a molecular weight range from 2.0 to 6.5 kDa with an average size of 3.5 kDa. In LAL and rFC assays, Peptide X completely inhibits endotoxin detection, even preventing recovery of defined endotoxin spikes. Preliminary experiments determined that the inhibitory effect is caused by tight binding of the peptide to endotoxin. This binding could not be displaced by salt, detergent, ultrafiltration, or heating. This mechanism of inhibition also causes extensive dilution to be insufficient. To demonstrate the extent of endotoxin inhibition by Peptide X, dilutions of the peptide in water were spiked with 1 EU/ml endotoxin and tested with the PyroGene® assay for spike recovery (Figure 1A). Starting with a concentration of 0.1 mg/ml, the first several 10-fold dilutions still showed inhibition at or near 100%. Dilution to 0.1 µg/ml was required to detect over 50% of endotoxin. For full endotoxin recovery, the peptide had to be diluted to a concentration of 10 ng/ml, which represents a 2,000,000-fold dilution of the therapeutic dose. This type of dilution prevents the detection of endogenous endotoxin.

Protein X was treated with the EndoPrep™ sample preparation kit to remove this inhibitory activity. A 0.1 mg/ml sample of Peptide X was prepared in BDTI™ Digestion Buffer and tested for PPC recovery with and without the EndoPrep™ protease composition treatment. Consistent with the above results, the PPC of the untreated sample was 100% inhibited (Figure 1B). Treatment with the EndoPrep™ protease composition increased PPC recovery to over 63% without incubation in the 37°C water bath. Proper incubation further increased the recovery to over 75%. This level of PPC recovery is in excess of the 50% required by the FDA to be considered an acceptable assay. To test this further, the ability of the EndoPrep™ protease composition to allow detection of contaminating endotoxin was examined. New samples of 0.1 mg/ml Peptide X were prepared in BDTI™ Digestion Buffer containing approximately 250 EU/mg endotoxin. Similar to the PPC results, treatment with the EndoPrep™ protease composition increased detection from 0 EU/ml to more than 150 EU/ml over the course of treatment (Figure 1C and Table 1). This represents the detection of over 60% of the calculated value of initial endotoxin added. PAGE analysis showed that the increase in endotoxin detection corresponded to the degradation of Peptide X (Figure 1D). EndoPrep™ protease composition addition without incubation reduced the amount of peptide by approximately 25-30%. Proper incubation further reduced the amount of native peptide to less than 10% after 30 minutes of treatment and almost negligible amounts after 120 minutes.

**Table 1. Fluorescence data at 60 minute PyroGene® (Lonza; Walkersville, MD) incubation.**

| **[Pepsin] EndoPrep™** | **Digestion Time** | **Measured endotoxin Content** | **1 EU/ml PPC Recovery** |
|---|---|---|---|
| 0% | 0' | 0 EU/ml | 0 EU/ml |
| 0.5% | 0' | 75 EU/ml | 0.631 EU/ml |
| 0.5% | 30' | 136 EU/ml | 0.755 EU/ml |
| 0.5% | 60' | 140 EU/ml | 0.726 EU/ml |
| 0.5% | 120' | 154 EU/ml | 0.652 EU/ml |

In addition to testing Peptide X digestions with the PyroGene® assay, they were also tested with the PyroGent® Gel Clot Assay from Lonza (Walkersville, MD) to demonstrate that the EndoPrep™ protease composition was applicable to more than one type of endotoxin detection system. The assay had a sensitivity of 0.06 EU/ml and was performed on untreated and treated 0.1 mg/ml peptide samples both with and without a 0.1 EU/ml PPC spike. All of the control reactions tested as expected. The 0.1 mg/ml peptide sample did not cause a clot formation either with or without the EndoPrep™ protease composition treatment indicating low endogenous endotoxin (Table 2). When the untreated peptide sample contained a 0.1 EU/ml PPC spike, which is in excess of the sensitivity level of the assay, there was no clot formation indicating that the endotoxin was masked by the peptide. When the treated peptide sample was spiked with a 0.1 EU/ml PPC there was clot formation indicating that the inhibitory factor was removed by digestion and the assay was detecting an endotoxin level in excess of 0.06 EU/ml.

**Table 2: EndoPrep™ treated samples in PyroGent® Gel Clot Assay**

| **Peptide (mg/ml)** | **EndoPrep™ Treatment** | **Spike (EU/ml)** | **Clot Formation** |
|---|---|---|---|
| 0.1 | No | - | No |
| 0.1 | No | 0.1 | No |
| 0.1 | Yes | - | No |
| 0.1 | Yes | 0.1 | Yes |

### Example 2: Sushi 3 Peptide

Factor C is the enzyme that initiates the enzymatic cascade in LAL and rFC assays. The endotoxin binding site of Factor C has been isolated to a 34 amino acid peptide termed Sushi 3 (S3). S3 is known to bind tightly to endotoxin. (Tan et al., FASEB J. 14:1801-13 (2000) via both electrostatic and hydrophobic interactions (Li et al., Biochemistry 45:10554-62 (2006); Li et al., Biochem. Soc. Trans. 34:270-2 (2006)). This binding is so strong it has been developed and is commercially available for use in endotoxin removal media (Ding et al., B. Biomed. Sci. Appl. 759:237-46 (2001)). Much like Peptide X, the tight interaction of S3 with endotoxin masks activity as detected in commonly used assays. Results of PPC inhibition tests (Figure 2A) demonstrate the requirement for extensive dilution of the peptide to prevent masking of 1 EU/ml. For complete PPC recovery, the peptide must be diluted to a concentration of 0.1 µg/ml. As with Peptide X, dilution of this extent prevents accurate endotoxin detection or quantitation.

To test the ability of the EndoPrep™ protease composition to alleviate the inhibitory effect of S3, a 0.1 mg/ml sample was prepared in BDTI™ Digestion Buffer from a 1 mg/ml stock solution. Previous experiments showed that the peptide contained no detectable endotoxin so exogenous endotoxin was added to the sample to a final concentration of 70 EU/mg. The control sample that received no EndoPrep™ protease composition tested at 17 EU/ml indicating over 75% inhibition (Figure 2B). Treatment with the EndoPrep™ protease composition recovered 29 EU/ml of endotoxin activity after a short incubation and 51 EU/ml after 120 minutes of incubation. This final sample represents a 300% increase in detection accuracy. The PAGE analysis corresponds to endotoxin recovery (Figure 2C). There is significant degradation after 15-30 minutes of incubation followed by further degradation with the 60 minute sample. After 120 minutes of treatment the peptide is almost undetectable.

### Example 3: Hemoglobin

The previous examples involved cationic peptides which caused complete or near complete endotoxin masking. However, the EndoPrep™ protease composition can also be used to increase the accuracy of endotoxin detection in protein samples that do not exhibit such dramatic effects.

Hemoglobin is the metalloprotein in erythrocytes responsible for delivering oxygen throughout the body. It has a moderate affinity for endotoxin (kD 3.1 x 10⁻⁸) as shown with ultrafiltration, density centrifugation, ethanol precipitation, and non-denaturing PAGE (Roth and Kaca, Artif. Cells Blood Substit. Immobil. Biotechnol. 22:387-98 (1994); Kaca et al., J. Biol. Chem. 269:25078-84 (1994)). Given its importance in biology and its potential role in artificial blood development, accurate endotoxin detection in hemoglobin is of great importance.

To test the effect of the EndoPrep™ protease composition treatment on hemoglobin (bovine erythrocyte), a 1 mg/ml sample was prepared in BDTI™ Digestion Buffer from a 50 mg/ml stock solution and treated as previously described. The untreated sample, containing no EndoPrep™ protease composition, gave a value of 330 EU/mg (Figure 3A). After treatment with the EndoPrep™ protease composition for 30 minutes, the measured endotoxin value increased nearly 70% to 560 EU/mg. This increase in activity continued over the 120 minute time course with results of 650 EU/mg at 60 minutes and 850 EU/mg after 120 minutes. This final data point represents an increase in measured endotoxin of over 150%. Accompanying the graph are the PAGE results of the samples indicating the extent of hemoglobin digestion over the time course (Figure 3B). In addition to the 16 kDa monomer, hemoglobin forms 32 kDa dimers and 64 kDa tetramers that can be visualized in reducing conditions. The extent of protein digestion can be seen by comparing the untreated sample in the first lane to the sample in the second lane which contains both hemoglobin and the EndoPrep™ protease composition but was not incubated in the water bath. Here, there is already significant reduction in all three hemoglobin populations. After 30 minutes of incubation at 37°C both the dimer and tetramer populations are completely digested. In addition, the monomer population is decreased by over 75%. After treatment for 120 minutes the monomer population is further reduced to less than 10% of the starting material.

These results show that as hemoglobin is digested, there is an accompanying increase in endotoxin activity. This pattern indicates that hemoglobin inhibits the detection of endotoxin. This is in contrast to some reports in the literature which claim an enhancing effect from hemoglobin (Roth and Kaca, Artif. Cells Blood Substit. Immobil. Biotechnol. 22:387-98 (1994); Kaca et al., J. Biol. Chem. 269:25078-84 (1994); Roth, Blood 83:2860-5 (1994); Roth et al., Transfusion 33:919-24 (1993)). One explanation given for the enhancing effect is that hemoglobin has a detergent-like activity which reduces LPS aggregate size and allows the LPS monomers to be more biologically available. However, most endotoxin deaggregating molecules, including melittin, lysozyme, complement proteins, BPI, and polymyxin B, cause a decrease in activity. It has also been suggested that enhancement may be due to phospholipid peroxidation, which would only be possible under certain assay criteria. This type of modification is not possible with the assay and may explain why some reports indicate inhibition while other publications report enhancement. Also, protein concentration and assay methods have been shown to be contributing factors in conflicting results involving protein effect on endotoxin (Kaca et al., J. Biol. Chem. 269:25078-84 (1994)). Regardless of the direction of effect, the above protocol would remove the hemoglobin from the sample and allow a more accurate determination of endotoxin content.

### Example 4: Bovine Serum Albumin

Albumin is the most abundant circulatory protein at a concentration of approximately 50 mg/ml (0.75 mM). It is involved in the delivery of fatty acids, hormones, bilirubin, bile salts, metals, and drugs throughout the body. It consists of three homologous domains, two of which have an exposed hydrophobic pocket thought to bind the Lipid A portion of the endotoxin molecule (David, Endotoxin in Health and Disease (ed. Brade, H.). New York:Marcel-Dekker, Inc., 413-22 (1999)). This is consistent with other reports suggesting that human serum albumin binds Lipid A via a non-electrostatic interaction (Jurgens et al., J. Endotoxin. Res. 8:115-26 (2002).

To test the effect of albumin on endotoxin detection a 100 mg/ml stock solution of bovine serum albumin (BSA) was prepared in endotoxin-free water. From this stock a 1 mg/ml sample was prepared in BDTI™ Digestion Buffer. Previous experiments determined that the endogenous endotoxin contamination of the BSA stock was very low (about 0.05 EU/mg) so exogenous endotoxin was added to a final concentration of 100 EU/mg. This sample was allowed to sit at room temperature for 30 minutes before the assay to allow potential interactions between the contents to be established. The untreated sample, containing no EndoPrep™ protease composition, gave a value of 58 EU/ml (Figure 4A), over 40% less than the calculated amount added. After treatment with the EndoPrep™ protease composition for 15 minutes, the measured endotoxin value increased to 67 EU/ml. At this same time point the PAGE data indicate extensive BSA digestion (Figure 4B). Increases in digestion time increased detectable endotoxin levels to as much as 77 EU/ml. This indicates recovery of nearly 80% of the known contaminating endotoxin and an increase in accuracy of over 30% compared to untreated sample.

There is no consensus on the effects of albumin on endotoxin detection in the literature. Studies have shown that albumin contributes to the solubility of the Lipid A portion of endotoxin to increase pyrogenicity and gelation in an LAL assay (Rietschel et al., Infect. Immun. 8:173-7 (1973)). Studies with recombinant human serum albumin (rHSA) show that the protein causes a change in micelle formation from a cubic form, which is typically moderately toxic, to a non-lamellar structure, indicating an increase in activity. However, this change in micelle structure caused minimal change in cytokine induction. In addition, tests with rHSA have shown complex changes in LAL coagulation assays without any discernible pattern (Jurgens et al., J. Endotoxin. Res. 8:115-26 (2002)). Similar reports with purified HSA have indicated enhancement activity with chromogenic LAL tests but no effect in gelation LAL assays (Kaca et al., J. Biol. Chem. 269:25078-84 (1994)). Other groups have found no effect of HSA on endotoxin activity as determined by either colorimetric or kinetic turbidimetric LAL assays or with the rabbit pyrogen test (Asakawa et al., Yakugaku. Zasshi. 114:888-93 (1994)). The current tests indicate a slight inhibitory effect of BSA on endotoxin detection. Regardless, as with hemoglobin, these tests show complete degradation of the protein and increased detection accuracy without negative effects on the assay system.

### Example 5: IgG

IgG is the most abundant immunoglobulin accounting for approximately 75% of all serum antibodies. It is a tetrameric molecule consisting of two g heavy chains (about 50 kDa each) and two light chains (about 23 kDa each). Each heavy chain is linked to a light chain through disulfide bonding. Likewise, the heavy chains are linked together with disulfide bonds giving the molecule the signature "Y" structure. The number of antibody therapies has risen greatly in the last decade and account for more than 400 ongoing preapproval clinical studies (Dubel, Appl. Microbiol. Biotechnol. 74:723-9 (2007)). Indications include arthritis, Crohn's disease, transplantation technology, psoriasis, autoimmunity, RSV, multiple sclerosis, and asthma. This is in addition to the field of cancer therapeutics which currently has promising antibody candidates for leukemia, mesothelioma, lymphoma, and colon, breast, kidney, lung, uterine, and pancreatic cancers (Dubel, Appl. Microbiol. Biotechnol. 74:723-9 (2007); Creput et al., Curr. Opin. Mol. Ther. 9:153-9 (2007); Rasmussen et al., Biotechnol. Lett. 29:845-52 (2007); Nayeem and Khan, Curr. Protein Pept. Sci. 7:165-70 (2006)). One reason for the increase in such therapies is the advent of recombinant antibodies. While this is a promising technology it demands a greater emphasis and sensitivity to endotoxin removal and detection.

The literature contains several reports indicating that IgG binds and neutralizes endotoxin. TNFα expression in response to endotoxin is reduced in the presence of IgG with a corresponding decrease in mortality in rats (Yentis et al., Cytokine 6:247-54 (1994); Xuan et al., Chemotherapy 47:194-202 (2001)). IgG has also been shown to neutralize free endotoxin in a dose-dependent method as assayed with a chromogenic LAL assay (Xuan et al., Antimicrob. Agents Chemother. 41:1512-6 (1997)). To test the application of the EndoPrep™ protease composition on the inhibitory activity of IgG a 0.5 mg/ml sample of rabbit IgG (from plasma) was prepared in BDTI™ Digestion Buffer from a 12.5 mg/ml stock solution. Previous studies showed low endotoxin contamination so exogenous endotoxin was added to a final concentration of approximately 100 EU/ml. This solution was allowed to sit at room temperature for 30 minutes before the assay to allow potential interactions between IgG and endotoxin to form. Assay results from the untreated IgG sample showed the recovery of 81 EU/ml (Figure 5A). Treatment with the EndoPrep™ protease composition increased endotoxin activity to as much as 123 EU/ml, an increase of over 50%. PAGE analysis showed a corresponding change in IgG structure (Figure 5B). In reduced conditions, the heavy and light chains run as distinct bands at approximately 53 and 23 kDa respectively. With the addition of the EndoPrep™ protease composition there was a decrease in the heavy chain population and corresponding appearances of a large fragment at 25-30 kDa and small Fc fragments near the bottom of the gel. In agreement with most reports in the literature, these results indicated that IgG has an inhibitory effect on endotoxin activity. This effect was removed with the EndoPrep™ protease composition treatment.

Each endotoxin molecule contains long hydrophilic sugar chains, hydrophobic fatty acid chains, and negatively charged phosphate groups. Because of these properties, endotoxin is known to bind a wide variety of proteins. This binding is known to change the micelle and/or aggregate structure and result in changes in activity. Because accurate endotoxin detection is of the utmost importance in the clinical setting, sensitive methods of detection are paramount. Described herein is the use of the Endo-Prep™ protease composition sample preparation system to remove the effects of proteins from samples containing endotoxin. Included have been examples of whole proteins and small peptides. In addition, the detection of both endogenous and exogenous endotoxin has been exhibited. In all examples shown, treatment with the EndoPrep™ protease composition increased the accuracy of endotoxin quantitation using either classical LAL or rFC assays (Table 3). The extent of change with the EndoPrep™ protease composition was dependent on the protein being tested. For example, the recovery of a large endotoxin spike was completely inhibited by untreated Peptide X but was recoverable following the EndoPrep™ protease composition treatment. Conversely, BSA only had a small inhibitory effect on endotoxin recovery. However, this effect was also removed with the EndoPrep™ protease composition treatment.

**Table 3: Summary of Results**

| | | | |
|---|---|---|---|
| Summary of the effects of the EndoPrep™ protease composition treatment on various proteins and peptides are shown. The table includes the amount of endotoxin determined experimentally in the samples before treatment and the peak recovery after treatment. The increase in detection is given as percent increase in endotoxin recovery. | | | |

| **Sample** | **Detection Before Treatment (EU/ml)** | **Detection After Treatment (EU/ml)** | **Increase in Detection** |
|---|---|---|---|
| Peptide X | <0.01 | 154 | >15,000% |
| Sushi 3 Peptide | 17 | 54 | 200% |
| Hemoglobin | 330 | 850 | 158% |
| BSA | 58 | 77 | 33% |
| IgG | 81 | 123 | 52% |

### Example 6: Use of pepsin to increase sensitivity of methods of detecting endotoxin.

The following experiments compared a series of LPS dilutions in water to a similar series containing a defined amount of pepsin. These data show pepsin enhanced the activity of the rFC Enzyme in the Lonza PyroGene® assay and resulted in an increase in sensitivity. This allowed LPS detection below the current 0.01 EU/ml LPS concentration.

**Pepsin Stock Preparation.** To create a 5% stock solution of pepsin (EndoPrep™ protease composition), 250 mg of pepsin were dissolved in 5 ml of digestion buffer (20 mM sodium acetate, pH 4.5). The mixture was vortexed until all the pepsin went into solution. The 5% pepsin solution was stored at 4°C.

**LPS Sample Preparation.** To create a series of LPS dilutions, a Lonza® (Lonza; Walkersville, MD) LPS stock (20 EU/ml) was serially diluted. A 10 EU/ml LPS solution was created by diluting 200 µl of water with 200 µl of Lonza® stock for a final volume of 400 µl. A 1 EU/ml LPS solution was created by adding 50 µl of the 10 EU/ml LPS solution to 450 µl of water for a final volume of 500 µl. A 0.1 EU/ml LPS solution was created by adding 50 µl of the 1 EU/ml LPS solution to 450 µl of water for a final volume of 500 µl. A 0.01 EU/ml LPS solution was created by adding 50 µl of a 0.1 EU/ml LPS solution to 450 µl of water for a final volume of 500 µl.

**LPS and pepsin Sample Preparation.** To create the same series of LPS dilutions that also contain 0.05% pepsin, two stock solutions of pepsin were made. A 0.1% stock solution of pepsin was made by adding 196 µl of water to 4 µl of 5% pepsin for a final volume of 200 µl. A 0.05% stock solution of pepsin was made by adding 1782 µl of water with 18 µl of 5% pepsin for a final volume of 1800 µl. 200 µl of the 0.1% pepsin solution was added to 200 µl of Lonza® LPS stock (20 EU/ml) to give 400 µl of a 10 EU/ml LPS, 0.05% pepsin solution. The 0.05% stock solution of pepsin was divided equally (450 µl) into 4 tubes. A 1 EU/ml LPS, 0.05% pepsin solution was created by adding 50 µl of the 10 EU/ml LPS, 0.05% pepsin solution to 450 µl of a 0.05% pepsin solution. A 0.1 EU/ml LPS, 0.05% pepsin solution was created by adding 50 µl of the 1 EU/ml LPS, 0.05% pepsin solution to 450 µl of a 0.05% pepsin solution. A 0.01 EU/ml LPS, 0.05% pepsin solution was created by adding 50 µl of the 0.1 EU/ml LPS, 0.05% pepsin solution to 450 µl of a 0.05% pepsin solution. A 0.001 EU/ml LPS, 0.05% pepsin solution was created by adding 50 µl of the 0.01 EU/ml LPS, 0.05% pepsin solution to 450 µl of a 0.05% pepsin solution.

**PyroGene® assay.** To test the endotoxin content of the samples prepared above, the PyroGene® assay was performed according to the manufacturer's specifications. Briefly, 50 µl of each sample was mixed with 50 µl of Pyrogene® working reagent (20 µl rFC assay buffer, 5 µl rFC enzyme solution, and 25 µl fluorogenic substrate) and was added to a single well of a Corning® (Coming Incorporated Life Sciences; Lowell, MA) 3604 Assay Plate. The plate was incubated in a BioTek Synergy 2 Plate Reader (BioTek; Winooski, VT) at 37°C and fluorescence readings were taken at 15, 30, 45, and 60 minute time points. Results are shown in Table 4 and in Figure 6.

**Table 4. Fluorescence data at 60 minute PyroGene® incubation.**

| **Sample** | **Fluorescence** |
|---|---|
| 10 EU/ml in water | 1,012,941 |
| 1 EU/ml in water | 214,209 |
| 0.1 EU/ml in water | 67,374 |
| 0.01 EU/ml in water | 52,604 |
| 10 EU/ml in water with 0.05% pepsin | 3,161,479 |
| 1 EU/ml in water with 0.05% pepsin | 586,139 |
| 0.1 EU/ml in water with 0.05% pepsin | 140,712 |
| 0.01 EU/ml in water with 0.05% pepsin | 83,678 |
| 0.001 EU/ml in water with 0.05% pepsin | 76,873 |
| Water | 52,096 |

Data were gathered for serial dilutions of untreated and treated LPS samples. Samples were treated with 0.05% pepsin. Treated samples were more sensitive to recombinant Factor C as evidenced by the increase in fluorescence.

Examples 1-6 demonstrated that acidic proteases can be used to enhance endotoxin detection in samples containing molecules that normally inhibit endotoxin detection. The molecules that normally inhibit endotoxin detection include a variety of different classes of peptides, such as short cationic peptides (i.e., Peptide X), enzymes (i.e., Sushi 3 peptide), metalloproteins (i.e., hemoglobin), circulatory proteins (i.e., albumin), and immunoglobulins (i.e., IgG). The examples included whole proteins and small peptides. Additionally, the detection of endogenous and exogenous endotoxin was demonstrated. In all examples shown, treatment with the EndoPrep™ protease composition increased the accuracy of endotoxin detection.

## Claims

1. A method for detecting gram negative bacteria or lipopolysaccharide in a sample comprising the steps of:
(a) contacting the sample with an active acidic protease under conditions that result in degradation of the proteins in the sample;
(b) inactivating the acidic protease;
(c) contacting the sample with one or more lipopolysaccharide binding polypeptides in the presence of an inactive acidic protease; and
(d) determining whether the one or more lipopolysaccharide binding polypeptides bind to the sample, binding indicating the sample contains gram negative bacteria or lipopolysaccharide, wherein the one or more lipopolysaccharide binding polypeptides are lipopolysaccharide binding polypeptides of an amebocyte lysate.

2. The method of claim 1, wherein the acidic protease is selected from the group consisting of pepsin, rennin, chymosin, plasmepsin, cathepsin D, cathepsin E, human urinary acid protease, HIV protease, *Neurospora oryzae* protease, *Mucor pusillus* protease, *Mucor miehei* protease, *Aspergillus niger* protease, *Rhizopus chinensis* protease, *Endothia parasitica* protease, yeast proteinase A, aspergillopepsinogen, rhizopuspepsin, penicillopepsin, and endothiapepsin.

3. The method of claim 2, wherein the amebocyte lysate is selected from the group consisting of lysates of *Limulus polyphemus, Tachypleus tridentatus, Carcinoscorpius rotundicauda* and *Tachypleus gigas.*

4. The method of claim 1, wherein the sample is a biological sample.

5. The method of claim 4, wherein the biological sample is selected from the group consisting of plasma, blood, serum, urine and saliva.

6. Use in a method of any one of claims 1-5 of a kit for detecting gram negative bacteria or lipopolysaccharide in a sample comprising one or more lipopolysaccharide binding polypeptides and an acidic protease, wherein the one or more lipopolysaccharide binding polypeptides are lipopolysaccharide binding polypeptides of an amebocyte lysate.

7. The use of claim 6, wherein the amebocyte lysate is selected from the group consisting of lysates of *Limulus polyphemus, Tachypleus tridentatus, Carcinoscorpius rotundicauda* and *Tachypleus gigas.*

8. The use of claim 6, wherein the acidic protease is selected from the group consisting of pepsin, rennin, chymosin, plasmepsin, cathepsin D, cathepsin E, human urinary acid protease, HIV protease, *Neurospora oryzae* protease, *Mucor pusillus* protease, *Mucor miehei* protease, *Aspergillus niger* protease, *Rhizopus chinensis* protease, *Endothia parasitica* protease, yeast proteinase A, aspergillopepsinogen, rhizopuspepsin, penicillopepsin, and endothiapepsin.

## Patentansprüche

1. Verfahren zum Nachweis Gram-negativer Bakterien oder Lipopolysacchariden in einer Probe, umfassend die Schritte:
(a) Inkontaktbringen der Probe mit einer aktiven sauren Protease unter Bedingungen, die Abbau der Proteine in der Probe zur Folge haben;
(b) Inaktivieren der sauren Protease;
(c) Inkontaktbringen der Probe mit einem oder mehreren Lipopolysaccharid-bindenden Polypeptiden in Gegenwart einer inaktiven sauren Protease; und
(d) Bestimmen, ob das eine oder die mehreren Lipopolysaccharid-bindenden Polypeptide an die Probe binden, wobei Bindung anzeigt, dass die Probe Gram-negative Bakterien oder Lipopolysaccharide enthält, wobei das eine oder die mehreren Lipopolysaccharid-bindenden Polypeptide Lipopolysaccharid-bindende Polypeptide eines Amöbozytenlysats sind.

2. Verfahren nach Anspruch 1, wobei die saure Protease ausgewählt ist aus der Gruppe bestehend aus Pepsin, Rennin, Chymosin Plasmepsin, Cathepsin D, Cathepsin E, menschlicher urinaler saurer Protease, HIV-Protease, *Neurospora oryzae* Protease, *Mucor pusillus* Protease, *Mucor miehei-*Protease*, Aspergillus niger* Protease, *Rhizopus chinensis* Protease, *Endothia parasitica* Protease, Hefe-Proteinase A, Aspergillopepsinogen, Rhizopuspepsin, Penicillopepsin, und Endothiapepsin.

3. Verfahren nach Anspruch 2, wobei das Amöbozytenlysat ausgewählt ist aus der Gruppe bestehend aus Lysaten von *Limulus polyphemus, Tachypleus tridentatus, Carcinoscorpius rotundicauda* und *Tachypleus gigas.*

4. Verfahren nach Anspruch 1, wobei die Probe eine biologische Probe ist.

5. Verfahren nach Anspruch 4, wobei die biologische Probe ausgewählt ist aus der Gruppe bestehend aus Plasma, Blut, Serum, Urin und Speichel.

6. Verwendung in einem Verfahren nach einem der Ansprüche 1-5 eines Kits zum Nachweis Gram-negativer Bakterien oder Lipopolysacchariden in einer Probe, das ein oder mehrere Lipopolysaccharid-bindende Polypeptide und eine saure Protease aufweist, wobei die ein oder mehreren Lipopolysaccharid-bindenden Polypeptide Lipopolysaccharid-bindende Polypeptide eines Amöbozytenlysats sind.

7. Verwendung nach Anspruch 6, wobei das Amöbozytenlysat ausgewählt ist aus der Gruppe bestehend aus Lysaten von *Limulus polyphemus, Tachypleus tridentatus, Carcinoscorpius rotundicauda* und *Tachypleus gigas.*

8. Verwendung nach Anspruch 6, wobei die saure Protease ausgewählt ist aus der Gruppe bestehend aus Pepsin, Rennin, Chymosin Plasmepsin, Cathepsin D, Cathepsin E, menschlicher urinaler saurer Protease, HIV-Protease, *Neurospora oryzae* Protease, *Mucor pusillus* Protease, *Mucor miehei* Protease, *Aspergillus niger* Protease, *Rhizopus chinensis* Protease, *Endothia parasitica* Protease, Hefe-Proteinase A, Aspergillopepsinogen, Rhizopuspepsin, Penicillopepsin, und Endothiapepsin.

## Revendications

1. Procédé de détection de bactéries Gram négatif ou d'un lipopolysaccharide dans un échantillon comprenant les étapes de :
(a) mise en contact de l'échantillon avec une protéase acide active dans des conditions qui conduisent à la dégradation des protéines dans l'échantillon ;
(b) inactivation de la protéase acide ;
(c) mise en contact de l'échantillon avec un ou plusieurs polypeptides de liaison de lipopolysaccharide en présence d'une protéase acide inactive ; et
(d) détermination si les un ou plusieurs polypeptides de liaison de lipopolysaccharide se lient à l'échantillon, la liaison indiquant que l'échantillon contient des bactéries Gram négatif ou un lipopolysaccharide, où les un ou plusieurs polypeptides de liaison de lipopolysaccharide sont des polypeptides de liaison de lipopolysaccharide d'un lysat d'amoebocytes.

2. Procédé de la revendication 1, dans lequel la protéase acide est choisie dans le groupe constitué des pepsine, rennine, chymosine, plasmepsine, cathepsine D, cathepsine E, protéase acide urinaire humaine, protéase du VIH, protéase de *Neurospora oryzae,* protéase de *Mucor pusillus,* protéase de *Mucor miehei,* protéase d'*Aspergillus niger,* protéase de *Rhizopus chinensis,* protéase d'*Endothia parasitica,* protéinase de levure A, aspergillopepsinogène, rhizopuspepsine, pénicillopepsine et endothiapepsine.

3. Procédé de la revendication 2, dans lequel le lysat d'amoebocytes est choisi dans le groupe constitué de lysats de *Limulus polyphemus, Tachypleus tridentatus, Carcinoscorpius rotundicauda* et *Tachypleus gigas.*

4. Procédé de la revendication 1, dans lequel l'échantillon est un échantillon biologique.

5. Procédé de la revendication 4, dans lequel l'échantillon biologique est choisi dans le groupe constitué du plasma, du sang, du sérum, de l'urine et de la salive.

6. Utilisation dans un procédé de l'une quelconque des revendications 1 à 5 d'un kit de détection de bactéries Gram négatif ou de lipopolysaccharide dans un échantillon comprenant un ou plusieurs polypeptides de liaison de lipopolysaccharide et une protéase acide, où les un ou plusieurs polypeptides de liaison de lipopolysaccharide sont des polypeptides de liaison de lipopolysaccharide d'un lysat d'amoebocytes.

7. Utilisation de la revendication 6, dans laquelle le lysat d'amoebocytes est choisi dans le groupe constitué de lysats de *Limulus polyphemus, Tachypleus tridentatus, Carcinoscorpius rotundicauda* and *Tachypleus gigas.*

8. Utilisation de la revendication 6, dans laquelle la protéase acide est choisie dans le groupe constitué des pepsine, rennine, chymosine, plasmepsine, cathepsine D, cathepsine E, protéase acide urinaire humaine, protéase du VIH, protéase de *Neurospora oryzae,* protéase de *Mucor pusillus,* protéase de *Mucor miehei,* protéase *d'Aspergillus niger,* protéase de *Rhizopus chinensis,* protéase *d'Endothia parasitica,* protéinase de levure A, aspergillopepsinogène, rhizopuspepsine, pénicillopepsine et endothiapepsine.
